# FASCICULE DE BREVET EUROPEEN

(11) **EP 2 556 853 B1**
(45) Date de publication et mention de la délivrance du brevet: **08.07.2015**
(21) Numéro de dépôt: 12290249.7
(22) Date de dépôt: 24.07.2012
(51) Int. Cl.: A61M 5/36, A61M 5/40

(54) **Dispositif avertisseur de fin de perfusion**
Warnvorrichtung zum Anzeigen, dass eine Infusion zu Ende geht
Warning device for end of drip

(30) Priorité: 01.08.2011 FR 1102399
(43) Date de publication de la demande: 13.02.2013
(73) Titulaire: INT' AIR MEDICAL, 01000 Bourg-en-Bresse (FR)
(72) Inventeur: David, François, 75016 Paris (FR)
(74) Mandataire: Flavenot, Bernard

(56) Documents cités:
- FR-A1- 2 321 904
- FR-A1- 2 711 531
- US-A- 3 105 490
- US-A1- 2008 077 116

## Description

La présente invention concerne les dispositifs avertisseurs de fin, ou de fin proche, d'une perfusion d'un fluide quand ce fluide est contenu dans une poche à paroi souple comportant au moins une tubulure de sortie et se présentant sous une forme gonflée quand elle est pleine de dit fluide et une forme aplatie quand elle est au moins partiellement vide.

Il est déjà connu de tels dispositifs avertisseurs de fin, ou de fin proche, d'une perfusion, comme par exemple celui qui est décrit et illustré dans le FR-A-2 321 904 qui concerne un dispositif pour la surveillance automatique d'une perfusion ou d'une instillation continue, qui comporte, à proximité immédiate de la tubulure de perfusion ou d'instillation, un détecteur produisant, en cas d'absence de liquide dans la tubulure, un signal de déclenchement pour, d'une part un dispositif d'alarme, et d'autre part des moyens d'obstruction de la tubulure en aval du détecteur. Un tel dispositif connu est en général assez complexe et parfois difficile à utiliser par le personnel soignant.

Aussi, la présente invention a-t-elle pour but de réaliser un dispositif avertisseur de fin, ou de fin proche, d'une perfusion, qui ait une structure compacte relativement simple à réaliser et qui soit, notamment pour le personnel soignant, d'une utilisation beaucoup plus facile que les avertisseurs de l'art antérieur.

Plus précisément, la présente invention a pour objet un dispositif avertisseur de fin, ou de fin proche, d'une perfusion d'un fluide quand ledit fluide est contenu dans une poche à paroi souple comportant au moins une tubulure de sortie et se présentant sous une forme gonflée quand elle est pleine de dit fluide et une forme aplatie quand elle est au moins partiellement vide, ayant les caractéristiques énoncées dans la revendication 1 annexée.

D'autres caractéristiques et avantages de la présente invention apparaîtront au cours de la description suivante donnée en regard des dessins annexés à titre illustratif mais nullement limitatif, dans lesquels :

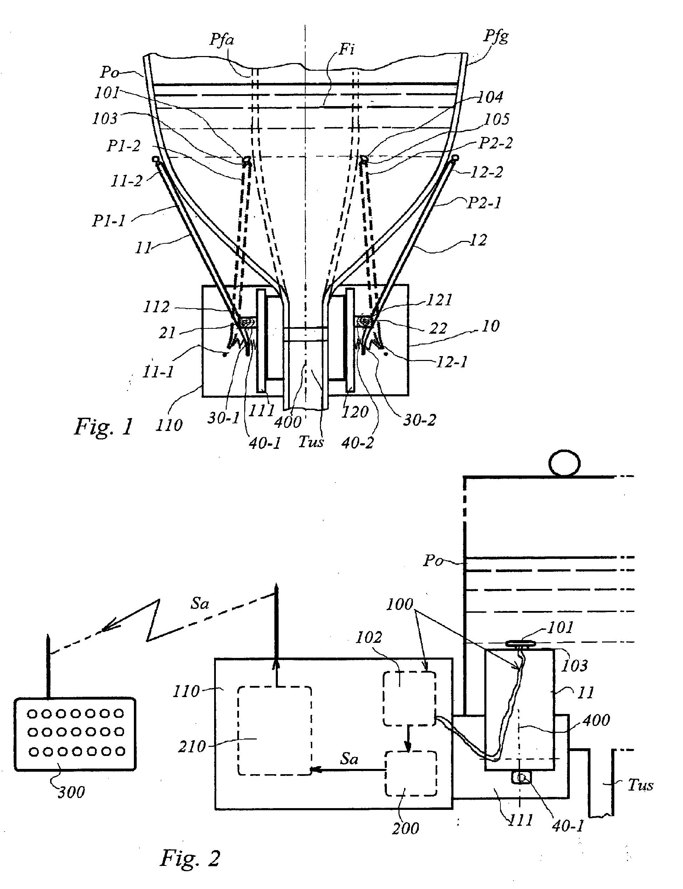

Les figures 1 et 2 représentent, respectivement vu de côté et vu de face, le schéma de principe d'un mode de réalisation du dispositif selon l'invention avertisseur de fin, ou de fin proche, d'une perfusion.

Il est tout d'abord précisé que, sur les figures, les mêmes références désignent les mêmes éléments, quelle que soit la figure sur laquelle elles apparaissent et quelle que soit la forme de représentation de ces éléments.

Il est aussi précisé que, dans la présente description, si l'adverbe "sensiblement" est associé à un qualificatif d'un moyen donné, ce qualificatif doit être compris au sens strict ou approché.

Le dispositif selon l'invention concerne un avertisseur de fin d'une perfusion d'un fluide Fi quand le fluide est contenu dans une poche Po à paroi souple comportant une tubulure de sortie Tus et se présentant sous une forme gonflée quand elle est pleine ou relativement pleine de ce fluide, et une forme aplatie ou relativement aplatie quand elle est au moins partiellement vide ou totalement vide.

Par "fin de perfusion", on entend également, dans le cadre de la présente description, "fin proche de perfusion". Ce qui signifie que l'expression "fin de perfusion" traduit l'état de la perfusion aussi bien lorsque la poche est totalement vide, que, de façon plus avantageuse, lorsqu'elle contient encore une certaine quantité de fluide Fi qui va permettre de continuer la perfusion pendant encore un certain temps avant que la poche ne soit totalement vide, et ce pour le bien du patient en permettant à la personne soignante d'avoir un laps de temps correct pour remplacer la poche avant qu'elle ne soit totalement vide, par une autre poche pleine.

Le dispositif comporte une embase 10 et deux pattes 11, 12. Dans la description donnée ci-après, il est utilisé l'expression "première et seconde pattes" éventuellement accompagnée des références 11 et 12. Mais il est bien évident que cette expression ne doit en aucun cas être prise pour qualifier de "première" plus particulièrement l'une des deux pattes par rapport à l'autre. Autrement dit, la première patte peut être la patte référencée 11, aussi bien que la patte référencée 12, et vice versa.

Le dispositif comporte aussi des moyens pour monter une seconde des deux pattes 11, 12 en coopération avec l'embase 10, et des moyens 30-1 pour monter la première des deux pattes 11, 12 en rotation par rapport à cette embase 10 autour d'un premier axe de rotation 21, 22 situé de préférence à proximité d'une première extrémité 11-1; 12-1 de la première patte. Ce premier axe de rotation est sensiblement parallèle au plan contenant la seconde patte et est situé à une distance de cette seconde patte supérieure à l'épaisseur de la poche Po quand elle est dans sa forme aplatie Pfa. De plus, la première patte est montée rotative autour du premier axe 21, 22 de façon qu'elle soit apte à prendre toutes les positions entre une première position et une seconde position, la première position P1-1, P2-1 étant celle dans laquelle sa seconde extrémité 11-2, 12-2 opposée à sa première extrémité 11-1, 12-1 est à une distance de la seconde patte au moins égale à la valeur de l'épaisseur de la poche Po dans sa forme gonflée Pfg, la seconde position P1-2, P2-2 étant celle dans laquelle sa seconde extrémité 11-2, 12-2 est à une distance de la seconde patte sensiblement égale à la valeur de l'épaisseur de la poche dans sa forme aplatie Pfa.

Le dispositif comporte en outre des moyens 100 pour délivrer un signal dit "de seuil" quand la distance entre la seconde extrémité 11-2, 12-2 de la première patte et la seconde patte atteint une valeur minimale déterminée, et des moyens pour délivrer un signal d'alarme Sa en fonction du signal de seuil.

Le dispositif peut comporter en outre des moyens 40-1, 40-2 pour exercer une force élastique sur la première patte pour tendre à l'amener dans sa seconde position P1-2, P2-2, par exemple, comme illustré, un ressort monté comprimé entre la première extrémité 11-1, 12-1 de la première patte et l'embase, figure 1, de façon que, en tendant à se détendre, il tend à la faire pivoter dans le sens dextrorsum et à l'amener vers sa position P1-2 représentée en traits interrompus sur cette figure 1.

Cependant, ces moyens de ressort 40-1, 40-2, sont relativement difficiles à tarer car leur tarage dépend de différents paramètres : nature de la matière dans laquelle est réalisée la poche, dimensions de cette dernière, température ambiante, etc. En conséquence, de façon préférentielle, le dispositif peut comporter, éventuellement en combinaison avec ces moyens de ressort 40-1, 40-2, mais avantageusement sans eux, des moyens pour coller la seconde extrémité 11-2, 12-2 d'au moins l'une des deux pattes 11, 12 sur la poche Po, avantageusement les deux, par exemple une bande adhésive double-face collée sur les pattes 11, 12 et sur la poche Po au moment de l'utilisation de l'avertisseur.

Dans le mode de réalisation décrit ci-dessus, il est prévu qu'une des deux pattes soit montée pivotante par rapport à l'embase 10 et que l'autre patte soit fixe par rapport à cette même embase 10.

Mais, de façon préférentielle, comme illustré sur la figure 1, les moyens pour monter l'autre patte, dénommée ici la seconde des deux pattes 11, 12, en coopération avec l'embase 10 sont constitués par des moyens 30-2 pour monter cette seconde patte en rotation par rapport à l'embase 10 autour d'un second axe de rotation 21, 22 situé à proximité de sa première extrémité 11-1, 12-1, ce second axe de rotation étant sensiblement parallèle au plan contenant la première patte et étant situé à une distance de cette première patte, lorsque cette dernière est dans une position par exemple intermédiaire entre ses première et seconde positions P1-1, P1-2, supérieure à l'épaisseur de la poche Po quand elle est dans sa forme aplatie Pfa, la seconde patte étant en outre montée rotative autour du second axe 21, 22 de façon qu'elle soit apte à prendre toutes les positions entre une troisième position et une quatrième position, la troisième position P1-1, P2-1 étant celle dans laquelle sa seconde extrémité 11-2, 12-2 opposée à sa première extrémité 11-1, 12-1 est à une distance de la première patte au moins égale à la valeur de l'épaisseur de la poche Po dans sa forme gonflée Pfg, la quatrième position P1-2, P2-2 étant celle dans laquelle cette seconde extrémité 11-2, 12-2 est à une distance de la première patte sensiblement égale à la valeur de l'épaisseur de la poche dans sa forme aplatie Pfa.

Quant aux moyens 100 pour délivrer un signal dit "de seuil" quand la distance entre la seconde extrémité 11-2, 12-2 de la première patte et la seconde patte atteint une valeur minimale déterminée, ils sont constitués par un circuit de détection comportant, montés en série, un détecteur ILS 101 et une source d'alimentation en énergie électrique 102 de ce détecteur ILS, des moyens 103 pour monter le détecteur ILS sur l'une des deux pattes 11, 12, un aimant 104, des moyens 105 pour monter cet aimant sur l'autre des deux pattes 11, 12 de façon qu'il n'active pas le détecteur ILS 101 lorsque les deux pattes sont éloignées l'une de l'autre (par exemple dans leurs positions P1-1 et P2-1), et qu'il active le détecteur ILS 101 lorsque les deux patte 11, 12 sont proches l'une de l'autre selon une valeur prédéterminée (par exemple dans leurs positions P1-2 et P2-2).

Un détecteur ILS est essentiellement composé d'un contact de type interrupteur à deux lames dont l'une au moins est souple (Interrupteur à Lame Souple). Lorsque l'aimant est éloigné du détecteur ILS, le contact de celui-ci est ouvert. Lorsque l'aimant, encore éloigné, s'approche du détecteur ILS, les deux lames sont soumises au champ magnétique généré par l'aimant. Chaque lame se comporte alors comme un aimant, mais la force d'attraction entre les deux lames n'est pas encore suffisante pour que la lame souple se déforme. En revanche, quand l'aimant est suffisamment proche du détecteur, la lame souple se déforme et entre en contact avec la lame rigide. Le contact électrique est alors établi et le courant électrique peut parcourir le circuit de détection défini ci-dessus.

Selon une réalisation avantageuse, l'embase 10 comporte un boîtier 110, une première plaque 111 en saillie sur un côté du boîtier 110, et un premier palier 112 monté sur une première face de la première plaque 111, ce premier palier étant centré sur le premier axe de rotation 21, 22 et la première patte étant alors montée rotative dans ce palier 112.

De façon encore plus préférentielle, l'embase 10 comporte en outre, figure 1, une seconde plaque 120 montée en coopération avec le boîtier 110, de préférence en saillie sur un côté du boîtier 110 (le même que celui qui porte la première plaque 111, ou un côté voisin) de façon qu'elle soit sensiblement parallèle à la première plaque 111 et en regard de la seconde face de la première plaque qui est opposée à sa première face, cette seconde plaque 120 étant en outre située à une distance de la première plaque 111 au moins égale à l'épaisseur de la poche Po dans sa forme aplatie Pfa, optionnellement au moins égale à l'épaisseur de la tubulure de sortie Tus de la poche. Un second palier 121 est monté sur la face de la seconde plaque qui est opposée à celle qui est en regard de la première plaque 111, ce second palier étant centré sur le second axe de rotation 21, 22 et la seconde patte étant alors montée rotative dans ce second palier.

Selon une réalisation possible qui peut présenter dans certains cas des avantages, la seconde plaque 120 est montée en rotation par rapport au boîtier via la première plaque 111 et un ressort en compression, autour d'un troisième axe de rotation 400 (schématiquement évoqué en traits-points sur les deux figures) sensiblement perpendiculaire au premier axe de rotation 21, 22, de façon à former pince avec la première plaque pour être apte par exemple à emprisonner un corps quelconque, notamment la tubulure de sortie Tus de la poche Po. Ce mode de réalisation peut en outre permettre de lier plus fixement le dispositif et la poche Po.

De façon préférentielle, les moyens pour délivrer un signal d'alarme Sa en fonction du signal de seuil sont constitués d'un capteur 200 monté en coopération avec le circuit de détection 100. Ce capteur, comme une bobine magnétique montée en coopération de façon connue sur le circuit, est apte à délivrer le signal d'alarme Sa lorsque le circuit de détection est parcouru par un courant électrique.

De façon très préférentielle, le dispositif comporte en outre des moyens de transmission 210 du signal d'alarme Sa selon au moins l'un des types de transmission suivants : filaire, hertzienne, optique, réseau internet, réseau intranet, vers par exemple une platine 300 qui peut se trouver dans le local du personnel soignant. Ce dernier sera alors averti comme décrit ci-après, que la perfusion est proche de sa fin et qu'il est temps d'intervenir.

La source d'alimentation en énergie électrique 102 du détecteur ILS, le capteur 200 et les moyens de transmission 210 seront avantageusement contenus dans le boîtier 110.

Le dispositif décrit ci-dessus et illustré sur les deux figures s'utilise et fonctionne de la façon suivante :
On suppose qu'une poche de perfusion Po est suspendue sur son support avec sa tubulure de sortie reliée au patient de façon bien connue en soit dans le domaine médical. L'utilisateur peut alors installer un dispositif selon l'invention en coopération avec cette poche Po. Pour cela, il amène les deux pattes dans leur première position P1-1 et P2-1 en appuyant sur leurs deux premières extrémités 11-1 et 12-1, leurs secondes extrémités 11-2 et 12-2 étant alors le plus éloignées l'une de l'autre, comme représenté en traits continus sur la figure 1. Si elles n'ont pas été pré-positionnées sur les pattes 11, 12, les bandes adhésives peuvent alors être placées entre les pattes et la poche.

Le dispositif est dans cette configuration positionné sur la partie basse de la poche Po, avec les deux plaques 111, 120 positionnées au niveau du fond de la poche et les deux pattes positionnées de chaque côté de la poche, comme illustré sur la figure 1. L'utilisateur peut alors relâcher la pression sur les premières extrémités des deux pattes, les secondes extrémités des deux pattes venant se plaquer contre la poche qui est gonflée de fluide Fi, et mettre le circuit de détection sous tension électrique.

Le dispositif reste accroché sur la poche grâce aux deux bandes adhésives. Cependant, par sécurité, il peut aussi être fixé par tout moyen au même support que celui sur lequel est accrochée la poche Po. Quand les deux plaques 111, 120 sont conformées en pince comme décrit ci-dessus, elles peuvent aussi être utilisées pour pincer la tubulure de sortie Tus de la poche Po.

Tant que la poche reste relativement remplie Pfg de fluide Fi, les secondes extrémités 11-2 et 12-2 des deux pattes sont relativement éloignées l'une de l'autre et l'aimant 104 n'active pas le détecteur ILS 101. Au fur et à mesure que la poche se vide et tend à prendre sa forme aplatie Pfa, l'aimant 104 et le détecteur ILS 101 se rapprochent l'un de l'autre, jusqu'à ce que, comme décrit ci-avant, le circuit de détection soit parcouru d'un courant électrique détecté par le capteur 200 qui émet un signal d'alarme Sa. L'instant où le circuit de détection est parcouru d'un courant électrique dépendra du réglage du détecteur ILS et de la puissance de l'aimant.

Le signal d'alarme est transmis à la salle de contrôle du personnel soignant, qui saura alors que la poche de perfusion est presque vide et qu'il faut sous peu intervenir pour la remplacer.

A la description ci-dessus, on constate que la structure du dispositif avertisseur de fin de perfusion selon l'invention est parfaitement compacte et que ce dispositif est très facile à utiliser et à mettre en oeuvre.

## Revendications

1. Dispositif avertisseur de fin d'une perfusion d'un fluide (Fi) quand ledit fluide est contenu dans une poche (Po) à paroi souple comportant une tubulure de sortie (Tus) et qui se présente sous une forme gonflée quand elle est pleine du dit fluide et une forme aplatie quand elle est au moins partiellement vide, **caractérisé par le fait qu'**il comporte :
• une embase (10)
• deux pattes (11, 12),
• des moyens pour monter une seconde des deux pattes (11, 12) en coopération avec ladite embase (10),
• des moyens (30-1) pour monter la première des deux pattes (11, 12) en rotation par rapport à ladite embase (10) autour d'un premier axe de rotation (21, 22) situé à proximité d'une première extrémité (11-1, 12-1) de cette première patte, ledit premier axe de rotation étant sensiblement parallèle au plan contenant ladite seconde patte et étant situé à une distance de cette dite seconde patte supérieure à l'épaisseur de la poche (Po) quand elle est dans sa forme aplatie (Pfa), ladite première patte étant montée rotative autour du premier axe (21, 22) de façon qu'elle soit apte à prendre toutes les positions entre une première position et une seconde position, la première position (P1-1, P2-1) étant celle dans laquelle sa seconde extrémité (11-2, 12-2) opposée à sa première extrémité (11-1, 12-1) est à une distance de la seconde patte au moins égale à la valeur de l'épaisseur de ladite poche (Po) dans sa forme gonflée (Pfg), la seconde position (P1-2, P2-2) étant celle dans laquelle sa seconde extrémité (11-2, 12-2) est à une distance de la seconde patte sensiblement égale à la valeur de l'épaisseur de ladite poche dans sa forme aplatie (Pfa),
• des moyens (100) pour délivrer un signal dit "de seuil" quand la distance entre la seconde extrémité (11-2, 12-2) de la première patte et la seconde patte atteint une valeur minimale déterminée, et
• des moyens pour délivrer un signal d'alarme (Sa) en fonction du dit signal de seuil.

2. Dispositif selon la revendication 1, **caractérisé par le fait que** les moyens pour monter la seconde des deux pattes (11, 12) en coopération avec ladite embase (10) sont constitués par des moyens (30-2) pour monter cette seconde patte en rotation par rapport à ladite embase (10) autour d'un second axe de rotation (21, 22) situé à proximité de sa première extrémité (11-1, 12-1), ce dit second axe de rotation étant sensiblement parallèle au plan contenant la première patte et étant situé à une distance de cette dite première patte, lorsque cette dernière est dans une position intermédiaire entre ses première et seconde positions (P1-1, P1-2), supérieure à l'épaisseur de la poche (Po) quand elle est dans sa forme aplatie (Pfa), la seconde patte étant montée rotative autour du second axe (21, 22) de façon qu'elle soit apte à prendre toutes les positions entre une troisième position et une quatrième position, la troisième position (P1-1, P2-1) étant celle dans laquelle sa,seconde extrémité (11-2, 12-2) opposée à sa première extrémité (11-1, 12-1) est à une distance de la première patte au moins égale à la valeur de l'épaisseur de ladite poche (Po) dans sa forme gonflée (Pfg), la quatrième position (P1-2, P2-2) étant celle dans laquelle cette seconde extrémité (11-2, 12-2) est à une distance de la première patte sensiblement égale à la valeur de l'épaisseur de ladite poche dans sa forme aplatie (Pfa).

3. Dispositif selon l'une des revendications précédentes, **caractérisé par le fait que** les moyens (100) pour délivrer un signal dit "de seuil" quand la distance entre la seconde extrémité (11-2, 12-2) de la première patte et la seconde patte atteint une valeur minimale déterminée sont constitués par:
• un circuit de détection comportant, montés en série, un détecteur ILS (101) et une source d'alimentation en énergie électrique (102) du dit détecteur ILS,
• des moyens (103) pour monter ledit détecteur ILS sur l'une des deux pattes (11, 12),
• un aimant (104),
• des moyens (105) pour monter ledit aimant sur l'autre des deux pattes (11, 12) de façon que, lorsque les deux pattes sont éloignées l'une de l'autre, ledit aimant (104) n'active pas ledit détecteur ILS (101) et que, lorsque les deux pattes (11, 12) sont proches l'une de l'autre selon une valeur prédéterminée, ledit aimant (104) active ledit détecteur ILS (101).

4. Dispositif selon l'une des revendications précédentes, **caractérisé par le fait que** ladite embase (10) comporte :
• un boîtier (110),
• une première plaque (111) en saillie sur un côté du dit boîtier (110), et
• un premier palier (112) monté sur une première face de ladite première plaque (111), ledit premier palier étant centré sur ledit premier axe de rotation (21, 22).

5. Dispositif selon les revendications 2 et 4, **caractérisé par le fait que** ladite embase (10) comporte :
• une seconde plaque (120) montée en coopération avec ledit boîtier (10) de façon qu'elle soit sensiblement parallèle à ladite première plaque (111) et en regard de la seconde face de la première plaque qui est opposée à sa première face, cette seconde plaque (120) étant en outre située à une distance de la première plaque (111) au moins égale à l'épaisseur de ladite poche (Po) dans sa forme aplatie (Pfa) et optionnellement au moins égale à l'épaisseur de ladite tubulure (Tus) de sortie, et
• un second palier (121) monté sur la face de la seconde plaque qui est opposée à celle qui est en regard de la première plaque (111), ledit second palier étant centré ledit second axe de rotation (21, 22).

6. Dispositif selon l'une des revendications précédentes quand elle dépend de la revendication 3, **caractérisé par le fait que** les moyens pour délivrer un signal d'alarme (Sa) en fonction du dit signal de seuil sont constitués d'un capteur (200) monté en coopération avec ledit circuit de détection, ledit capteur étant apte à délivrer ledit signal d'alarme (Sa) lorsque ledit circuit de détection est parcouru par un courant électrique.

7. Dispositif selon la revendication 6, **caractérisé par le fait qu'**il comporte en outre des moyens de transmission (210) du signal d'alarme (Sa) selon au moins l'un des types de transmission suivants : filaire, hertzienne, optique, réseau internet, réseau intranet.

8. Dispositif selon l'une des revendications précédentes, **caractérisé par le fait qu'**il comporte en outre des moyens pour coller la seconde extrémité d'au moins l'une des dites pattes (11, 12) sur ladite poche (Po).

9. Dispositif selon l'une des revendications précédentes, **caractérisé par le fait qu'**il comporte des moyens (40-1, 40-2) pour exercer une force élastique sur ladite première patte (11, 12) pour tendre à l'amener dans sa seconde position (P1-2, P2-2).

10. Dispositif selon l'une des revendications précédentes quand elle dépend de la revendication 5, **caractérisé par le fait que** la seconde plaque (120) est montée en rotation par rapport au boîtier (110) via la première plaque (111) et un ressort en compression autour d'un troisième axe de rotation (400) sensiblement perpendiculaire au dit premier axe de rotation (21, 22) de façon à former pince avec cette première plaque (111).

## Patentansprüche

1. Vorrichtung zum Warnen vor dem Ende der Perfusion eines Fluids (Fi), wenn das Fluid in einer Tasche (Po) mit nachgiebigen Wänden enthalten ist, die einen Auslassschlauch (Tus) besitzt und eine aufgeblasene Form aufweist, wenn sie mit dem Fluid gefüllt ist, und eine abgeflachte Form aufweist, wenn sie wenigstens teilweise leer ist, **gekennzeichnet durch**:
• eine Basis (10),
• zwei Laschen (11, 12),
• Mittel, um eine zweite der beiden Laschen (11, 12) in Zusammenwirkung mit der Basis (10) zu montieren,
• Mittel (30-1), um die erste der beiden Laschen (11, 12) drehbar in Bezug auf die Basis (10) um eine erste Drehachse (21, 22), die sich in der Nähe eines ersten Endes (11-1, 12-1) dieser ersten Lasche befindet, zu montieren, wobei die erste Drehachse zu der Ebene, die die zweite Lasche enthält, im Wesentlichen parallel ist und sich in einem Abstand von dieser zweiten Lasche befindet, der größer als die Dicke der Tasche (Po) ist, wenn sie ihre abgeflachte Form (Pfa) aufweist, wobei die erste Lasche drehbar um die erste Achse (21, 22) in der Weise montiert ist, dass sie alle Positionen zwischen einer ersten Position und einer zweiten Position einnehmen kann, wobei die erste Position (P1-1, P2-1) jene ist, in der sich ihr zweites Ende (11-2, 12-2) gegenüber ihrem ersten Ende (11-1, 12-1) in einem Abstand von der zweiten Lasche befindet, der wenigstens gleich dem Wert der Dicke der Tasche (Po) in ihrer aufgeblasenen Form (Pfg) ist, und wobei die zweite Position (P1-2, P2-2) jene ist, in der sich ihr zweites Ende (11-2, 12-2) in einem Abstand von der zweiten Lasche befindet, der im Wesentlichen gleich dem Wert der Dicke der Tasche in ihrer abgeflachten Form (Pfa) ist,
• Mittel (100) zum Ausgeben eines so genannten "Schwellenwert"-Signals, wenn der Abstand zwischen dem zweiten Ende (11-2, 12-2) der ersten Lasche und der zweiten Lasche einen bestimmten minimalen Wert erreicht, und
• Mittel zum Ausgeben eines Alarmsignals (Sa) als Funktion des Schwellenwert-Signals.

2. Vorrichtung nach Anspruch 1, **dadurch gekennzeichnet, dass** die Mittel zum Montieren der zweiten der Laschen (11, 12) in Zusammenwirkung mit der Basis (10) durch Mittel (30-2) gebildet sind, um diese zweite Lasche drehbar in Bezug auf die Basis (10) um eine zweite Drehachse (21, 22), die sich in der Nähe ihres ersten Endes (11-1, 12-1) befindet, gebildet sind, wobei diese zweite Drehachse zu der Ebene, die die erste Lasche enthält, im Wesentlichen parallel ist und sich in einem Abstand von dieser ersten Lasche befindet, wenn diese Letztere in einer Zwischenposition zwischen der ersten und der zweiten Position (P1-1, P1-2) ist, der größer als die Dicke der Tasche (Po) ist, wenn diese in ihrer abgeflachten Form (Pfa) ist, wobei die zweite Lasche drehbar um die zweite Achse (21, 22) montiert ist, derart, dass sie alle Positionen zwischen einer dritten Position und einer vierten Position einnehmen kann, wobei die dritte Position (P1-1, P2-1) jene ist, in der sich ihr zweites Ende (11-2, 12-2) gegenüber ihrem ersten Ende (11-1, 12-1) in einem Abstand von der ersten Lasche befindet, der wenigstens gleich dem Wert der Dicke der Tasche (Po) in ihrer aufgeblasenen Form (Pfg) ist, wobei die vierte Position (P1-2, P2-2) jene ist, in der sich dieses zweite Ende (11-2, 12-2) in einem Abstand von der ersten Lasche befindet, der im Wesentlichen gleich dem Wert der Dicke der Tasche in ihrer abgeflachten Form (Pfa) ist.

3. Vorrichtung nach einem der vorhergehenden Ansprüche, **dadurch gekennzeichnet, dass** die Mittel (100) zum Ausgeben eines sogenannten "Schwellenwert"-Signals, wenn der Abstand zwischen dem zweiten Ende (11-2, 12-2) der ersten Lasche und der zweiten Lasche einen bestimmten minimalen Wert erreicht, gebildet sind durch:
• eine Detektionsschaltung, die in Reihe einen Detektor ILS (101) und eine Quelle für die Versorgung mit elektrischer Energie (102) des Detektors ILS enthält,
• Mittel (103) zum Montieren des Detektors ILS an einer der beiden Laschen (11, 12),
• einen Magneten (104),
• Mittel (105) zum Montieren des Magneten an der anderen der zwei Laschen (11, 12) in der Weise, dass dann, wenn die zwei Laschen voneinander entfernt sind, der Magnet (104) den Detektor ILS (101) nicht aktiviert, und dass dann, wenn die beiden Laschen (11, 12) entsprechend einem vorgegebenen Wert nahe beieinander sind, der Magnet (104) den Detektor ILS (101) aktiviert.

4. Vorrichtung nach einem der vorhergehenden Ansprüche, **dadurch gekennzeichnet, dass** die Basis (10) umfasst:
• ein Gehäuse (110),
• eine erste Platte (111), die auf einer Seite von dem Gehäuse (110) vorsteht, und
• ein erstes Lager (112), das an einer ersten Fläche der ersten Platte (111) montiert ist, wobei das erste Lager auf die erste Drehachse (21, 22) zentriert ist.

5. Vorrichtung nach den Ansprüchen 2 und 4, **dadurch gekennzeichnet, dass** der Sockel (10) umfasst:
• eine zweite Platte (120), die in Zusammenwirkung mit dem Gehäuse (10) in der Weise montiert ist, dass sie zu der ersten Platte (111) im Wesentlichen parallel ist und die sich über der zweiten Fläche der ersten Platte, die sich gegenüber der ersten Fläche befindet, befindet, wobei diese zweite Platte (120) sich außerdem in einem Abstand von der ersten Platte (111) befindet, der wenigstens gleich der Dicke der Tasche (Po) in ihrer abgeflachten Form (Pfa) ist und optional wenigstens gleich der Dicke des Auslassschlauchs (Tus) ist, und
• ein zweites Lager (121), das an der Fläche der zweiten Platte, die sich gegenüber jener befindet, die sich gegenüber der ersten Platte (111) befindet, montiert ist, wobei das zweite Lager auf die zweite Drehachse (21, 22) zentriert ist.

6. Vorrichtung nach einem der vorhergehenden Ansprüche, wenn abhängig von Anspruch 3, **dadurch gekennzeichnet, dass** die Mittel zum Ausgeben eines Alarmsignals (Sa) als Funktion des Schwellenwertsignals durch einen Sensor (200) gebildet sind, der in Zusammenwirkung mit der Detektionsschaltung montiert ist, wobei der Sensor das Alarmsignal (Sa) ausgeben kann, wenn die Detektionsschaltung von einem elektrischen Strom durchflossen wird.

7. Vorrichtung nach Anspruch 6, **dadurch gekennzeichnet, dass** sie außerdem Mittel (210) zum Übertragen des Alarmsignals (Sa) wenigstens gemäß einem der folgenden Übertragungstypen umfasst: drahtgebunden, funktechnisch, optisch, Internet-Netz, Intranet-Netz.

8. Vorrichtung nach einem der vorhergehenden Ansprüche, **dadurch gekennzeichnet, dass** sie außerdem Mittel umfasst, um das zweite Ende wenigstens einer der Laschen (11, 12) an die Tasche (Po) zu kleben.

9. Vorrichtung nach einem der vorhergehenden Ansprüche, **dadurch gekennzeichnet, dass** sie Mittel (40-1, 40-2) umfasst, um auf die erste Lasche (11, 12) eine elastische Kraft auszuüben, die bestrebt ist, sie in ihre zweite Position (P1-2, P2-2) zu führen.

10. Vorrichtung nach einem der vorhergehenden Ansprüche, wenn abhängig von Anspruch 5, **dadurch gekennzeichnet, dass** die zweite Platte (120) drehbar in Bezug auf das Gehäuse (110) über die erste Platte (111) und eine Kompressionsfeder um eine dritte Drehachse (400), die zu der ersten Drehachse (21, 22) im Wesentlichen senkrecht ist, montiert ist, derart, dass mit dieser ersten Platte (111) eine Zange gebildet wird.

## Claims

1. A warning device for issuing a warning concerning the end of a fluid drip when said fluid (Fi) is contained in a flexible-walled pouch (Po) having an outlet tube (Tus) and that is in an inflated shape when it is full of said fluid and in a flat shape when it is at least partially empty, the device being **characterized in that** it comprises:
· a base (10);
· two tabs (11, 12);
· means for mounting a second one of the two tabs (11, 12) in co-operation with said base (10);
· means (30-1) for mounting the first one of the two tabs (11, 12) to pivot relative to said base (10) about a first pivot axis (21, 22) situated in the proximity of a first end (11-1, 12-1) the first tab, said first pivot axis being substantially parallel to the plane containing said second tab and being situated at a distance from said second tab that is greater than the thickness of the pouch (Po) when it is in its flattened shape (Pfa), said first tab being mounted to pivot about the first axis (21, 22) so as to be suitable for occupying all positions between a first position and a second position, the first position (P1-1, P2-1) being that in which its second end (11-2, 12-2) remote form its first end (11-1, 12-1) is at a distance from the second tab that is not less than the value of the thickness of said pouch (Po) in its inflated shape (Pfg), the second position (P1-2, P2-2) being that in which its second end (11-2, 12-2) is at a distance from the second tab that is substantially equal to the value of the thickness of said pouch in its flattened shape (Pfa);
· means (100) for delivering a "threshold" signal when the distance between the second end (11-2, 12-2) of the first tab and the second tab reaches a determined minimum value; and
· means for delivering an alarm signal (Sa) as a function of said threshold signal.

2. A device according to claim 1, **characterized by** the fact that the means for mounting the second of the two tabs (11, 12) in co-operation with said base (10) are constituted by means (30-2) for mounting the second tab to pivot relative to said base (10) about a second pivot axis (21, 22) situated in the proximity of its first end (11-1, 12-1), said second pivot axis being substantially parallel to the plane containing the first tab and being situated at a distance from said first tab when it is in an intermediate position between its first and second positions (P1-1, P1-2) that is greater than the thickness of the pouch (Po) when it is in its flattened shape (Pfa), the second tab being mounted to pivot about the second axis (21, 22) so as to be suitable for occupying all positions between a third position and a fourth position, the third position (P1-1, P2-1) being the position in which its second end (11-2, 12-2) remote from its first end (11-1, 12-1) is at a distance from the first tab that is not less than the value of the thickness of said pouch (Po) in its inflated shape (Pfg), the fourth position (P1-2, P2-2) being the position in which said second end (11-2, 12-2) is at a distance from the first tab that is substantially equal to the value of the thickness of said pouch in its flattened shape (Pfa).

3. A device according to either preceding claim, **characterized by** the fact that the means (100) for delivering a "threshold" signal when the distance between the second end (11-2, 12-2) of the first tab and the second tab reaches a determined minimum value are constituted by:
· a detector circuit comprising, connected in series, a reed-switch detector (101) and an electrical power supply (102) for said reed-switch detector;
· means (103) for mounting said reed-switch detector on one of the two tabs (11, 12);
· a magnet (104); and
· means (105) for mounting said magnet on the other one of the two tabs (11, 12) in such a manner that, when the two tabs are spaced apart from each other, said magnet (104) does not activate said reed-switch detector (101), and that, when the two magnets (11, 12) are close to each other at a predetermined value, said magnet (104) activates said reed-switch detector (101).

4. A device according to any preceding claim, **characterized by** the fact that said base (10) comprises:
· a box (110);
· a first plate (111) projecting from one side of said box (110); and
· a first bearing (112) mounted on a first face of said first plate (111), said first bearing being centered on said first pivot axis (21, 22).

5. A device according to claims 2 and 4, **characterized by** the fact that said base (10) includes:
· a second plate (120) mounted to co-operate with said box (10) so as to be substantially parallel to said first plate (111) and facing the second face of the first plate that is opposite from its first face, said second plate (120) also being situated at a distance from the first plate (111) that is not less than the thickness of said pouch (Po) in its flattened shape (Pfa) and optionally not less than the thickness of said outlet tube (Tus); and
· a second bearing (121) mounted on the face of the second plate that is opposite from its face facing the first plate (111), said second bearing being centered on said second pivot axis (21, 22).

6. A device according to any preceding claim when dependent on claim 3, the device being **characterized by** the fact that the means for delivering an alarm signal (Sa) as a function of said threshold signal are constituted by a sensor (200) mounted to co-operate with said detector circuit, said sensor being suitable for delivering said alarm signal (Sa) when said detector circuit passes an electric current.

7. A device according to claim 6, **characterized by** the fact that it further includes transmitter means (210) for transmitting the alarm signal (Sa) using at least one of the following types of transmission: by wire, by radio, by light, by Internet, by Intranet.

8. A device according to any preceding claim, **characterized by** the fact that it further includes means for sticking the second end of at least one of said tabs (11, 12) to said pouch (Po).

9. A device according to any preceding claim, **characterized by** the fact that it includes means (40-1, 40-2) for exerting a resilient force on said first tab (11, 12) in order to tend to bring it into its second position (P1-2, P2-2).

10. A device according to any preceding claim, when dependent on claim 5, **characterized by** the fact that the second plate (120) is pivotally mounted relative to the box (110) via the first plate (111) and a compression spring around a third pivot axis (400) substantially perpendicular to said first pivot axis (21, 22) so as to co-operate with said first plate (111) to form a clamp.
